# EUROPÄISCHE PATENTANMELDUNG

(11) **EP 4 245 355 A1**
(43) Veröffentlichungstag der Anmeldung: **20.09.2023**
(21) Anmeldenummer: 22161748.3
(22) Anmeldetag: 13.03.2022
(51) Int. Cl.: A61M 60/109, A61M 60/81, A61M 60/824, A61M 60/825, A61M 60/804, F04D 29/046

(54) **FLUIDPUMPE**

(71) Anmelder: Berlin Heart GmbH, 12247 Berlin (DE)
(72) Erfinder: Richert, Hendryk, 12247 Berlin (DE); Peters, Oliver, 14129 Berlin (DE)
(74) Vertreter: Pfenning, Meinig & Partner mbB

(57) **Zusammenfassung**

Die vorliegende Erfindung betrifft eine Fluidpumpe zum Fördern eines Fluids, insbesondere von Blut. Die Fluidpumpe umfasst einen Fluidkanal, welcher von einer Kanalwand begrenzt wird und einen in dem Fluidkanal angeordneten Rotor, welcher um einen Drehpunkt des Lagers drehbar mittels eines mechanischen, hydrodynamischen und/oder hydrostatischen, axialen und radialen Lagers gelagert ist. Der Fluidkanal weist einen sphärischen Abschnitt auf. Der Rotor weist einen Rotorkörper und ein daran innerhalb des sphärischen Abschnitts des Fluidkanals angeordnetes Förderelement auf, welches geeignet ist, eine zumindest bereichsweise im Wesentlichen sphärische Rotationsfläche des Rotors zu erzeugen. Dabei fallen der sphärische Mittelpunkt des sphärischen Abschnitts des Fluidkanals und der sphärische Mittelpunkt der sphärischen Rotationsfläche mit dem Drehpunkt im Wesentlichen zusammen, sodass ein minimaler Abstand zwischen dem Rotor und der Kanalwand im sphärischen Abschnitt bei Verkippen des Rotors konstant ist.

## Beschreibung

Die vorliegende Erfindung betrifft eine Fluidpumpe zum Fördern eines Fluids, insbesondere von Blut.

Blutpumpen werden in verschiedenen Konfigurationen gebaut. Rotoren mechanisch gelagerter Blutpumpen werden meist front- und rückseitig mit Ball-Cup-Lagern gehalten. Ein axialer Spalt von wenigen Mikrometern in den Lagern erlaubt ein freies Drehen bei guter axialer Führung. Blut kann prinzipiell in die Lager diffundieren. Diese Lager entsprechen in der Mechanik zwei beabstandeten Festlagern. Durch Ablagerung von Proteinen im offenen Lagerspalt besteht die Gefahr, dass es zu erheblichen axialen Kräften in diesem Lager kommt. Selbst bei niedrigen Reibkoeffizienten führt die erhöhte Axialkraft zu erhöhten Reibleistungen, welche im schlimmsten Fall zu einer Koagulation der biologischen Bestandteile im und um das Lager führt, was zu einem schlechteren Reibbeiwert sowie einem schlechteren Wärmeabtransport in das Blut führt. Ein Versagen der Lager und damit der Pumpen ist dann eine Frage der Zeit. Aufgrund der schlechten Beherrschung der mechanischen Lager, insbesondere durch unvorhersagbare und weitestgehend unverstandene Thrombogenität, auch aufgrund des benannten Effektes, werden diese Pumpentypen nur vereinzelt weiterverfolgt.

Eine weitere Form der mechanischen Lagerung stellt eine Konus-Lagerung dar, wobei das mechanische Lager axial durchspült werden kann. Allerdings führt auch hier Ablagerung im Spalt zu einer Zunahme des Reibbeiwertes mit bereits aufgeführten potentiellen Konsequenzen.

Eine andere Entwicklungsrichtung beschäftigte sich mit hydrodynamisch gelagerten Pumpen. Hier wird Blut als Schmier- und Tragmittel genutzt. Die erhöhte Scherbelastung des Blutes im Spalt wird von diesem offenbar gut toleriert. Diese Systeme scheinen bei niedriger Hämolyse gut zu funktionieren, sind aber aufgrund der leicht erhöhten Thrombogenität weitestgehend aus der Anwendung verschwunden.

Vorteil all dieser Pumpen besteht in einer sehr einfachen Steuerung, da lediglich der Rotor angetrieben werden muss. Eine standardisierte Back-EMF-Motorsteuerung, die die Induktion in den Motorspulen auswertet, genügt für den normalen Betrieb. Dadurch kann auf Elektronik in der Pumpe verzichtet werden, und in der Driveline müssen minimal drei Leitungen vorhanden sein.

Demgegenüber stehen die vollmagnetisch gelagerten Pumpen. Diese Pumpengeneration zeichnet die vollständige Kontrolle der Rotorlage durch magnetische Kräfte aus und ermöglicht das kontaktfreie Schweben eines Rotors im Strömungskanal. Jedoch ist hierfür eine umfangreiche Steuerelektronik in der Pumpe notwendig. Die Driveline solcher Pumpen weist üblicherweise einen großen Durchmesser auf und ist sehr wenig flexibel. Ist die Steuerelektronik für das aktive Magnetlager in die Pumpe integriert, kann die Driveline zwar mit weniger Leitungen auskommen. Die Pumpenbasis ist allerdings vergrößert, was für kleinere Patienten zu einem Problem führen kann.

Als Zwischengattung mit Potential für den implantierbaren Bereich finden sich passiv magnetisch gelagerte Pumpen. Der Rotor wird hier beispielsweise axial frontseitig mechanisch gelagert und front- und auslassseitig magnetisch radial gefesselt. Die Steuerung des Rotors beschränkt sich wie bei den mechanisch gelagerten Pumpen auf die Rotation des Rotors. Die mechanische Lagerung besteht in einer reinen Axiallagerung, sodass der Rotor radial ausgelenkt wird, indem er den radial wirkenden Kräften entsprechend der Lagersteifigkeit des Magnetlagers folgt, d.h. der Rotor bewegt sich auf einer zykloiden Bahn um die Symmetrieachse der radialen magnetischen Lagerung der Pumpe. Da die Kräfte weitestgehend an den Schaufeln in der Volute angreifen, ist weiterhin davon auszugehen, dass der Rotor verkippen wird, sodass sich die axiale Auflagerfläche verringert. Das führt langfristig zu einer partiellen Abnutzung (Erhöhung der Rauigkeit) der Oberfläche im Bereich der vom Rotorlager überstrichenen Fläche. Zusätzlich kann Blut in das verkippte mechanische Lager eindringen und dort aktiviert, bzw. zermahlen werden. Es ist also davon auszugehen, dass hier eine thrombogene Gefahrenzone existiert.

Axialbeschaufelte magnetisch passiv gelagerte Blutpumpen haben den Nachteil, dass der Schaufelspalt durch die Rückstelleigenschaften der magnetischen Lager immer groß sein muss, wodurch die Schaufelverluste bei diesem Pumpentyp intrinsisch erhöht sind.

Pumpen, die auf die Rotorlagerregelung verzichten können, eignen sich tendenziell besser zur Miniaturisierung, enthalten weniger Komponenten, sind dadurch einfacher aufgebaut, also robuster und deutlich kleiner zu bauen als ein vollmagnetisches System.

Eine axiale Pumpe fördert im Niedrigflussbereich deutlich schlechter, wenn der Radialspalt zu groß wird, da die Spaltverluste (die Überströmung der Schaufelkanten entgegen der Hauptströmungsrichtung) überproportional ansteigen. Ebenfalls kritisch ist der Umgang mit fehlender Dämpfung in passiven magnetischen Lagern, die dazu führt, dass periodische Anregungen kleiner Amplitude in der Nähe der Resonanz zu großen Auslenkungen der Rotoren (im Millimeterbereich) führen können. Erfahrungsgemäß befindet sich eine Resonanz immer im Arbeitsbereich der Blutpumpen, da die Drehzahl patientenabhängig ist. Schaufeln können bei Berührung Beschädigungen der Wand erzeugen und damit eine verstärkte Blutschädigung bzw. Keimbildung zum Thrombenwachstum begünstigen.

Es ist daher eine Aufgabe der vorliegenden Erfindung eine axiale Fluidpumpe bereitzustellen, die die oben beschriebenen Probleme des Standes der Technik löst. Weiterhin ist es eine Aufgabe der vorliegenden Erfindung eine axiale Fluidpumpe bereitzustellen, die effizient, robust und sicher gegenüber radialer Verkippung des Rotors ist und eine Miniaturisierung ermöglicht.

Die oben beschriebene Aufgabe wird durch eine Fluidpumpe gemäß vorliegendem Anspruch 1 gelöst. Vorteilhafte Weiterbildungen der Erfindung werden in den abhängigen Ansprüchen beschrieben.

Eine erfindungsgemäße Fluidpumpe zum Fördern eines Fluids, insbesondere von Blut, umfasst einen Fluidkanal, welcher von einer Kanalwand begrenzt wird, einen in dem Fluidkanal angeordneten Rotor, welcher um einen Drehpunkt des Lagers drehbar mittels eines mechanischen, hydrodynamischen und/oder hydrostatischen, axialen und radialen Lagers gelagert ist, wobei der Fluidkanal einen sphärischen Abschnitt aufweist, der Rotor einen Rotorkörper und ein daran innerhalb des sphärischen Abschnitts des Fluidkanals angeordnetes Förderelement aufweist, welches geeignet ist, eine zumindest bereichsweise im Wesentlichen sphärische Rotationsfläche des Rotors zu erzeugen, und wobei der sphärische Mittelpunkt des sphärischen Abschnitts des Fluidkanals und der sphärische Mittelpunkt der sphärischen Rotationsfläche mit dem Drehpunkt im Wesentlichen zusammenfallen, sodass ein minimaler Abstand zwischen dem Rotor und der Kanalwand im sphärischen Abschnitt bei Verkippen des Rotors konstant ist.

Durch die sphärische Anordnung von sphärischem Abschnitt des Fluidkanals und Förderelement kann der Rotor sehr weit aus seiner nominellen Lage herauskippen, ohne dass es dabei zu einem Kontakt zwischen Rotor und Fluidkanal kommen kann. In diesem Fall kann der minimale Abstand zwischen Rotor und der Kanalwand des Fluidkanals ideal eingestellt werden. Durch die oben beschriebene sphärische Ausgestaltung und Anordnung von Fluidkanal und Rotor wird eine Berührung zwischen Rotor und Kanalwand vermieden, sodass auf zusätzliche Maßnahmen wie z.B. die Verwendung eines Sicherheitslagers verzichtet werden kann. Dadurch kann die Fluidpumpe einfach, kompakt und sicher gebaut werden.

Der Fluidkanal kann vorzugsweise hohlzylinderförmig und im Wesentlichen rotationssymmetrische um eine Drehachse ausgestaltet sein. Das Fluid strömt im Wesentlichen in einer Längsrichtung, welche einer Richtung entlang der Drehachse entspricht. Der sphärische Abschnitt des Fluidkanals befindet sich vorzugsweise im Bereich oder in der Nähe des Lagers. Insbesondere überlappt das Lager in Längsrichtung mit dem sphärischen Abschnitt oder ist benachbart, insbesondere direkt benachbart zu dem sphärischen Abschnitt angeordnet.

Eine sphärische hydrodynamische Lagerung bietet den Vorteil, dass die Lagerung ein Verkippen des Rotors ohne eine Beeinträchtigung der Lagerwirkung zulässt.

Gemäß einem vorteilhaften Ausführungsbeispiel der Erfindung kann das Lager ein Ball/Cup-Lager oder ein Pin-Lager, insbesondere ein Nadel-auf-Sackloch-Lager, sein.

Gemäß einem weiteren vorteilhaften Ausführungsbeispiel der vorliegenden Erfindung kann die Fluidpumpe weiter ein passiv magnetisches Lager umfassen, wobei das passiv magnetische Lager als Kipplager zur Rückstellung oder Begrenzung einer Verkippung des Rotors ausgebildet ist und/oder eingerichtet ist, den Rotor in Bezug auf den Fluidkanal axial vorzuspannen. Durch die Vorspannung kann verhindert werden, dass der Rotor zu stark ins Lager gedrückt wird und es dort zu Reibverlusten kommt. Außerdem lässt sich mittels der magnetischen Vorspannung ein idealer Lagerspalt einstellen, in welchen das zu fördernde Fluid, insbesondere Blut, nicht hineinfließen kann. Die magnetische Vorspannung kann ebenfalls dazu genutzt werden, den Rotor bei jedem Betriebszustand in das Lager zu drücken.

Gemäß einem weiteren vorteilhaften Ausführungsbeispiel der vorliegenden Erfindung kann die Fluidpumpe weiter einen an der Kanalwand des Fluidkanals angeordneten Motorstator und einen im Rotorkörper oder im Förderelement integrierten Motormagneten umfassen, wobei eine passiv magnetische Kipplagerung durch eine magnetische Anziehung oder Abstoßung zwischen dem Motorstator und dem Motormagneten realisiert wird.

Gemäß einem weiteren vorteilhaften Ausführungsbeispiel der vorliegenden Erfindung kann der Fluidkanal auf einer dem Lager gegenüberliegenden Seite konisch, sich zum Lager hin verjüngend geformt sein, wobei ein Verjüngungswinkel des Fluidkanals einem maximalen Verkippungswinkel des Rotors innerhalb des Fluidkanals entspricht. Eine konische Verjüngung des Fluidkanals zum Lager hin ermöglicht ein Anschmiegen des Rotors an den Fluidkanal und verhindert eine Berührung bzw. Beschädigung des Rotorkörpers bei einer starken radialen Verkippung des Rotors.

Gemäß einem weiteren vorteilhaften Ausführungsbeispiel der vorliegenden Erfindung kann der Rotorkörper auf einer dem Lager gegenüberliegenden Seite konisch, sich vom Lager weggerichtet verjüngend geformt sein, wobei ein Verjüngungswinkel des Rotorkörpers einem maximalen Verkippungswinkel des Rotors innerhalb des Fluidkanals entspricht. Eine konische Verjüngung des Rotors zum Einlassbereich hin ermöglicht ein Anschmiegen des Rotors an die Kanalwand und verhindert eine Berührung bzw. Beschädigung des Rotorkörpers im Einlassbereich bei einer starken radialen Verkippung des Rotors. Die konische Verjüngung des Rotors kann mit dem Fluidkanal ein großflächiges hydrodynamisches Lager bilden, welches in der Lage ist, einen Kontakt zwischen Rotor und Fluidkanal, auch bei Kippmomenten größer als dem maximalen Kippmoment des passiven Magnetlagers oder Motorstators, zu verhindern.

Gemäß einem weiteren vorteilhaften Ausführungsbeispiel der vorliegenden Erfindung kann die Fluidpumpe weiter ein im Fluidkanal angeordnetes hydrostatisches oder hydrodynamisches Hilfslager zur Begrenzung der Verkippung des Rotors umfassen.

Gemäß einem weiteren vorteilhaften Ausführungsbeispiel der vorliegenden Erfindung kann das hydrostatische oder hydrodynamische Hilfslager von an der Kanalwand angeordneten Nachleitschaufeln gebildet werden.

Gemäß einem weiteren vorteilhaften Ausführungsbeispiel der vorliegenden Erfindung können auf einer dem Lager gegenüberliegenden Seite des Fluidkanals an der Kanalwand und/oder am Rotor hydrodynamisch wirksame Elemente angeordnet sein, um bei Verkippung des Rotors ein Anschmiegen des Rotors an die Kanalwand zu verbessern.

Gemäß einem weiteren vorteilhaften Ausführungsbeispiel der vorliegenden Erfindung kann der Fluidkanal einen Fluideinlass und einen Fluidauslass aufweisen, und das Lager am Fluideinlass, am Fluidauslass oder in einer Mitte des Fluidkanals angeordnet sein.

Gemäß einem weiteren vorteilhaften Ausführungsbeispiel der vorliegenden Erfindung kann der Fluidkanal weiter einen axialen, tangentialen oder axialtangential-gemischten Fluidauslass aufweisen.

Gemäß einem weiteren vorteilhaften Ausführungsbeispiel der vorliegenden Erfindung kann der Fluidkanal einen Fluidauslass umfassen, und die Fluidpumpe im Bereich des Fluidauslasses eine Volute aufweisen, insbesondere eine Ringvolute, eine logarithmische Volute und/oder eine Volute mit einem axialen Anteil.

Gemäß einem weiteren vorteilhaften Ausführungsbeispiel der vorliegenden Erfindung kann das mechanische Lager ein hämokompatibles, hartes, abriebfestes und/oder wärmeleitfähiges Material, insbesondere Keramik, insbesondere Aluminiumoxid (Al₂O₃), Siliziumcarbid (SiC), Zirkoniumoxid (ZrO₂) oder Siliziumnitrid (Si₃N₄), Mischkeramik, insbesondere Al₂O₃/SiC, Aluminium-verstärktes Zirkoniumoxid (ATZ) oder Zirkoniumoxid-verstärktes Aluminiumoxid (ZTA), Kristallin, insbesondere Diamant, Saphir, Rubin oder Quarz, oder Tantalnitrid, insbesondere eine Tantalnitrid-Dünnschicht aufweisen oder daraus bestehen, und/oder eine Gleitschicht, insbesondere Diamond-Like Carbon (DLC), SiN oder Wolframcarbid/Kohlenstoff (WC/C) aufweisen.

Gemäß einem weiteren vorteilhaften Ausführungsbeispiel der vorliegenden Erfindung kann das Lager axial mechanisch verschiebbar sein, um einen idealen Abstand zwischen dem Fluidkanal und dem Förderelement einzustellen.

Gemäß einem weiteren vorteilhaften Ausführungsbeispiel der vorliegenden Erfindung kann das Lager vor Inbetriebnahme der Fluidpumpe mittels eines Gewindes axial mechanisch verschiebbar sein.

Gemäß einem weiteren vorteilhaften Ausführungsbeispiel der vorliegenden Erfindung können das magnetische Lager und/oder der Motorstator axial verschiebbar sein, sodass die Vorspannung des Rotors im Lager einstellbar ist.

Gemäß einem weiteren vorteilhaften Ausführungsbeispiel der vorliegenden Erfindung können unter der sphärischen Rotationsfläche des Rotors Motor- oder Lagermagnete angeordnet sein.

Gemäß einem weiteren vorteilhaften Ausführungsbeispiel der vorliegenden Erfindung können die Magnete einen geschlossenen oder durchbrochenen Ring bilden, welcher die Förderelemente verbindet.

Gemäß einem besonders bevorzugten Ausführungsbeispiel führt eine Fluidpumpe mit wenigstens partiell sphärischem Fluidkanal im Bereich des Lagers und der Förderelemente somit zu einer Lösung, bei der der Rotor in einem Punkt axial, sowie radial mit Hilfe eines einzigen mechanischen Ball-Cup-Lagers oder mithilfe eines hydrodynamischen oder hydrostatischen Lagers fixiert wird. Die verbleibenden drei Freiheitsgrade (Rotation, bzw. Kippen um zwei Achsen) werden durch magnetische Kräfte kontrolliert. Die Rotation um die Drehachse wird durch einen Motor bestimmt. Die Kippfreiheitsgrade werden passiv durch (i) ein zusätzliches Magnetlager oder (ii) die Interaktion zwischen Motormagneten und Motorstator gefesselt. Durch diese sphärische Anordnung kann der Rotor sehr weit aus seiner nominellen Lage herauskippen, ohne dass es dabei zu einem Kontakt zwischen Förderelement und Kanalwand kommen kann. Der Spalt zwischen Förderelement und Kanalwand kann auf das strömungstechnisch ideale Maß eingestellt werden, da der Rotor in den drei geometrisch entscheidenden Freiheitsgraden mechanisch oder hydraulisch präzise gelagert ist. Verluste der Förderelemente werden minimiert und retrograder Fluss durch die Pumpe wird durch die steile Kennlinie vermieden.

Das magnetisch vorgespannte mechanische Lager kann nicht durch axiale Verspannung gegen ein zweites, unnachgiebiges mechanisches Lager zu einer Erhöhung der Reibleistung führen, sodass die Lagerverlustleistung dauerhaft auf einem niedrigen Niveau verbleibt. Die Koagulation von Blutbestandteilen wird so vermieden und das Lager bleibt langfristig funktionsfähig. Der vergrößerte erlaubte Kippbereich des Rotors ist für die Magnetlagerung vorteilhaft, da die bei Axialpumpen zu geringe Fluiddämpfung nun genügt, eine Berührung zwischen Rotor und Kanalwand zu verhindern. Der Aufbau mit einem stützenden Ringmagnetlager ermöglicht eine optimale Durchspülung der Fluidpumpe mit nur geringen Sekundärstromanteilen im Bereich des mechanischen Lagers. Der Aufbau lässt prinzipiell ein frontseitiges bzw. rückseitiges mechanisches Auflager zu, wobei die Kombination des auslassseitigen Lagers mit einer Volute besonders vorteilhaft sein kann. Eine hydrodynamische oder hydrostatische Lagerung erscheinen ebenso möglich. Der Motor und die Lagermagnete können im Rotorkörper aber auch in den Förderelementen oder in einer Außensphäre untergebracht sein.

Das vorgeschlagene Fluidpumpendesign bietet die Vorteile, dass eine (über die Kanten der Förderelemente hinausgehende) Sekundärströmung verhindert wird, dass der Motortreiber und damit die Steuereinheit einfach ist, da keine aktive Lagerregelung notwendig ist und der Motor als Wärmequelle im beschleunigten Hauptstrom gekühlt werden kann. Weiterhin ist kein Fanglager mehr notwendig, da sich der Rotor frei bis zum potentiellen Aufgleiten des Rotors, insbesondere bei der konischen Ausgestaltung des Rotors, auf die Kanalwand auslenken kann. Die Kennlinie der Fluidpumpe ist besonders einfach einstellbar und die mechanischen Lagerkomponenten können besonders einfach belastungs- und strömungsoptimiert gestaltet werden. Der entscheidende Vorteil bezüglich der Laufstabilität ist, dass die Turbulenzen stromabwärts der Förderelemente auf keinen Rotor treffen und diesen damit nicht zum Schwingen anregen können.

Im Folgenden werden einige Beispiele einer erfindungsgemäßen Fluidpumpe anhand von Figuren detaillierter beschrieben. Dabei werden verschiedene erfindungswesentliche oder auch vorteilhafte weiterbildende Elemente im Rahmen dieser Beispiele genannt, wobei auch einzelne dieser Elemente als solche zur Weiterbildung der Erfindung - auch herausgelöst aus dem Kontext des jeweiligen Beispiels und weiterer Merkmale des jeweiligen Beispiels-verwendet werden können. Weiterhin werden in den Figuren für gleiche oder ähnliche Elemente gleiche oder ähnliche Bezugszeichen verwendet und deren Erläuterung daher teilweise weggelassen.

Es zeigen
- Figur 1: eine einlassseitig gelagerte Blutpumpe gemäß einem ersten Ausführungsbeispiel der vorliegenden Erfindung,
- Figur 2: eine einlassseitig gelagerte Blutpumpe gemäß einem zweiten Ausführungsbeispiel der vorliegenden Erfindung,
- Figur 3: eine einlassseitig gelagerte Blutpumpe gemäß einem dritten Ausführungsbeispiel der vorliegenden Erfindung,
- Figur 4: eine einlassseitig gelagerte Blutpumpe gemäß einem vierten Ausführungsbeispiel der vorliegenden Erfindung,
- Figur 5: eine auslassseitig gelagerte Blutpumpe gemäß einem fünften Ausführungsbeispiel der vorliegenden Erfindung,
- Figur 6: die auslassseitig gelagerte Blutpumpe gemäß aus Figur 5 mit Rotorverkippung,
- Figur 7: eine auslassseitig gelagerte Blutpumpe gemäß einem sechsten Ausführungsbeispiel der vorliegenden Erfindung,
- Figur 8: eine auslassseitig gelagerte Blutpumpe gemäß einem siebten Ausführungsbeispiel der vorliegenden Erfindung und
- Figur 9: eine auslassseitig gelagerte Blutpumpe gemäß einem achten Ausführungsbeispiel der vorliegenden Erfindung.

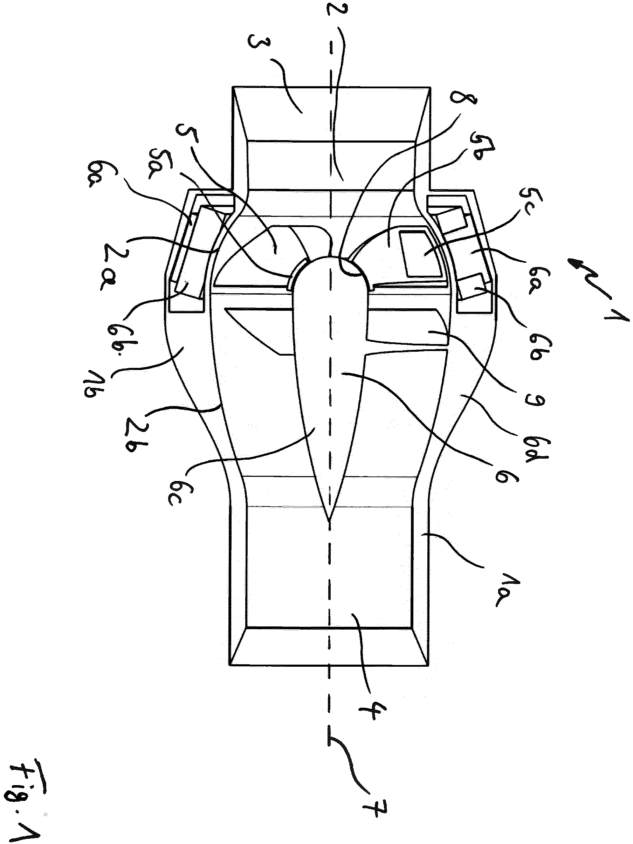

Figur 1 zeigt eine Blutpumpe 1 gemäß einem ersten Ausführungsbeispiel der vorliegenden Erfindung in einer Längsschnittansicht. Die Blutpumpe 1 weist ein im Wesentlichen hohlzylinderförmiges Pumpengehäuse 1a auf, welches im Wesentlichen rotationssymmetrisch um eine Drehachse 7 ist. In einem mittleren Bereich des Pumpengehäuses 1a weist das Pumpengehäuse 1a eine sphärische Aufweitung 1b auf. In einem inneren Bereich des Pumpengehäuses 1a weist die Blutpumpe 1 einen Fluidkanal 2 auf, der von einer Kanalwand 2b der Fluidpumpe 1 begrenzt wird. Der Fluidkanal 2 erstreckt sich in axialer Richtung von einem Einlassbereich 3 zu einem Auslassbereich 4. Die sphärische Aufweitung 1b befindet sich zwischen dem Einlassbereich 3 und dem Auslassbereich 4.

Im Bereich der sphärischen Aufweitung 1b weist der Fluidkanal 2 einen sphärischen Abschnitt 2a auf. In diesem Bereich weist die Blutpumpe 1 ferner einen Rotor 5 und einen Stator 6 auf. Der Stator 6 umfasst ein tropfenförmiges Element 6c, welches stromlinienförmig entlang der Drehachse 7 im Fluidkanal 2 angeordnet ist. Weiterhin ist das Pumpengehäuse 1a Teil des Stators 6. Das tropfenförmige Element 6c ist über Nachleitschaufeln 9, welche ebenfalls Teil des Stators 6 sind, mit dem Pumpengehäuse 1a fest verbunden. Im Pumpengehäuse 1a ist im Bereich der Aufweitung 1b eine Vielzahl von Statoreisen 6a ringförmig um den Fluidkanal 2 angeordnet. Die Statoreisen 6a sind jeweils von Statorwicklungen 6b parallel zur Kanalwand 2b umgeben.

Das vordere, einlassseitige Ende des tropfenförmigen Elements 6c bildet ein axial durchspülbares mechanisches Ball-Cup-Lager 8 für den Rotor 5. Der Rotor 5 ist drehbar um die Drehachse 7 radial und axial mechanisch auf dem tropfenförmigen Element 6c des Stators 6 gelagert. Der Rotor 5 wird von einem kleinen Rotorkörper 5a auf dem tropfenförmigen Element 6c gehalten. Der Rotorkörper 5a verbindet Förderelemente 5b, welche einen Hauptteil des Rotors 5 ausmachen, starr miteinander.

In einem äußeren, zur Kanalwand 2b hin gelegenen Bereich der Förderelemente 5b sind Permanentmagnete 5c in die Förderelemente 5b integriert. Die Statoreisen 6a und zugehörigen Statorwicklungen 6b bilden eine Vielzahl von Elektromagneten, die mit den Permanentmagneten 5c zusammenwirken, um den Rotor 5 anzutreiben. Weiterhin dienen die Elektromagneten 6a, 6b des Stators 6 zusammen mit den Permanentmagneten 5c des Rotors 5 einer passiven Rückstellung des Rotors 5 bei Verkippung des Rotors 5 in Richtung der Kanalwand 2b und damit einer Fixierung der verbleibenden zwei Kippfreiheitsgrade des Rotors 5.

Weiterhin ist auf zur Kanalwand 2b hin gelegenen Außenseiten eine Form der Förderelemente 5b an eine sphärische Krümmung der Kanalwand 2b angepasst, sodass die Förderelemente 5b bei Rotation des Rotors 5 eine sphärische Rotationsfläche überstreichen. Weiterhin sind die Lage des Rotors 5 im Fluidkanal 2 und die Position des sphärischen Abschnitts 2a derart zueinander ausgerichtet, dass der Mittelpunkt der sphärischen Rotationsfläche der Förderelemente 5b bzw. des Rotors 5 und der Mittelpunkt des sphärischen Abschnitts 2a des Fluidkanals 2 im Wesentlichen mit dem Drehpunkt des Lagers 8 zusammenfallen. Dadurch bleibt ein minimaler Abstand zwischen den Förderelementen 5b bzw. dem Rotor 5 und der Kanalwand 2b auch bei einer Verkippung des Rotors 5 zur Kanalwand 2b hin im Wesentlichen konstant. Dadurch wird eine Berührung des Rotors 5 mit der Kanalwand 2b und eine damit verbundene mögliche Beschädigung des Rotors 5 und Beeinträchtigung der Funktion der Blutpumpe 1 verhindert.

Die Förderelemente 5b dienen der Förderung des mit der Blutpumpe 1 zu fördernden Blutes vom Einlassbereich 3 zum Auslassbereich 4 und sind derart gestaltet, dass eine Förderung in einer gemischt radialen und axialen Richtung ermöglicht wird. Die Nachleitschaufeln 9 sorgen zusätzlich zu den Förderelementen 5b für eine effiziente Beförderung des Blutes zum Auslass 4 der Blutpumpe 1.

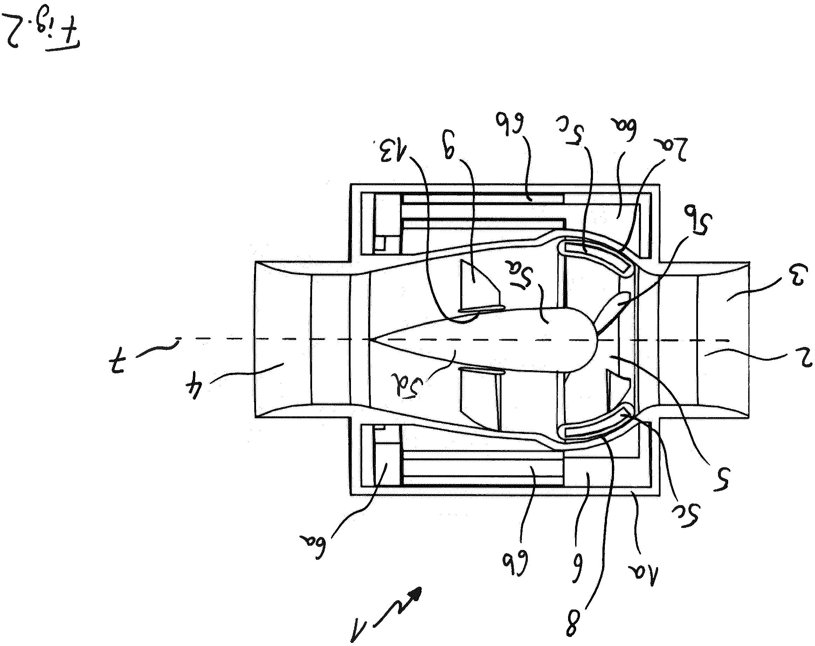

Figur 2 zeigt eine Blutpumpe 1 gemäß einem zweiten Ausführungsbeispiel der vorliegenden Erfindung in einer Längsschnittansicht. Die Blutpumpe 1 weist ein Pumpengehäuse 1a mit einem Einlassbereich 3 für das Blut und einem Auslassbereich 4 für das Blut auf. Das Pumpengehäuse 1a ist im Wesentlichen rotationssymmetrisch zu einer Drehachse 7. Durch das Pumpengehäuse 1a führt in axialer Richtung ein Fluidkanal 2, welcher von einer Kanalwand 2b begrenzt wird. Der Fluidkanal 2 weist zwischen dem Einlassbereich 3 und dem Auslassbereich 4 eine im Wesentlichen tropfenförmige Form auf. Stromabwärts des Einlassbereichs 3 weist der Fluidkanal 2 zunächst einen sphärischen Abschnitt 2a auf, bevor sich der Fluidkanal 2 in Richtung des Auslassbereichs 4 verjüngt.

In dem tropfenförmigen Bereich des Fluidkanals 2 ist ein Rotor 5 mit einem Rotorkörper 5a angeordnet. Im vorderen, dem Einlassbereich 3 zugewandten Bereich des Rotors 5 ist der Rotorkörper 5a ringförmig ausgestaltet und über Förderelemente 5b mit einem tropfenförmigen Element 5d, welches Teil des Rotorkörpers 5a ist, verbunden. Zum Auslassbereich 4 hin läuft das tropfenförmige Element 5d spitz zu. In dem ringförmigen Bereich ist eine Vielzahl von Permanentmagneten 5c angeordnet. Der Rotor 5 ist in dem sphärischen Abschnitt 2a radial und axial hydrodynamisch oder hydrostatisch gelagert, wobei die Kanalwand 2b im sphärischen Abschnitt das hydrodynamische bzw. hydrostatische Lager 8 darstellt. Weiterhin wird das tropfenförmige Element 5d des Rotors 5 mittels eines ringförmigen hydrodynamischen oder hydrostatischen Lagers 13 radial gelagert, wobei das hydrodynamische bzw. hydrostatische Lager 13 über Nachleitschaufeln 9 mit dem Pumpengehäuse 1a starr verbunden ist. Das Lager 8 fixiert den ringförmigen Bereich des Rotors 5 in axialer und radialer Richtung. Das Lager 13 fixiert das tropfenförmige Element 5d des Rotors 5 in radialer Richtung.

Die Blutpumpe 1 ist mit einem lagerlosen Motorstator zum Antreiben des Rotors 5 ausgestattet. Um den tropfenförmigen Bereich des Fluidkanals 2 herum sind im Pumpengehäuse 1a eine Vielzahl von Statoreisen 6a angeordnet. Die Statoreisen 6a sind im Bereich des sich verjüngenden Fluidkanals 2 senkrecht zur Kanalwand 2b mit Statorwicklungen 6b umwickelt. Im Bereich des sphärischen Abschnitts 2a liegen die Statoreisen 6a an der Außenseite des sphärischen Fluidkanals an. Die Statoreisen 6a mit Statorwicklungen 6b bilden Elektromagnete, die mit den Permanentmagneten 5c zusammenwirken, um den Rotor 5 anzutreiben.

Im sphärischen Abschnitt 2a des Fluidkanals 2 ist der ringförmige Bereich des Rotors 5 auf der zur Kanalwand 2b hin gelegenen Außenseite ebenfalls sphärisch geformt und an eine Krümmung der Kanalwand 2b angepasst. Weiterhin fallen der Mittelpunkt der sphärischen Krümmung der Außenseite des ringförmigen Bereichs des Rotors 5 und der Mittelpunkt der sphärischen Krümmung des sphärischen Abschnitts 2a im Wesentlichen mit dem Drehpunkt des Lagers 8 zusammen, sodass der minimale Abstand zwischen dem ringförmigen Bereich des Rotors 5 und der Kanalwand 2b auch bei einer Verkippung des Rotors 5 im Wesentlichen konstant ist. Dadurch wird eine Berührung des Rotors 5 mit der Kanalwand 2b und eine damit verbundene mögliche Beschädigung des Rotors 5 und Beeinträchtigung der Funktion der Blutpumpe 1 verhindert.

Die Förderelemente 5b dienen der Förderung des mit der Blutpumpe 1 zu fördernden Blutes vom Einlassbereich 3 zum Auslassbereich 4 und sind derart ausgestaltet, dass eine Förderung des Blutes in einer gemischt radialen und axialen Richtung ermöglicht wird. Die stromabwärts angeordneten Nachleitschaufeln 9 sorgen zusätzlich zu den Förderelementen 5b für eine effiziente Beförderung des Blutes zum Auslass der Blutpumpe 1.

Das Zusammenwirken von Rotormagneten 5c und Motorstator 6a, 6b begrenzt passiv eine radiale Verkippung des Rotors 5. Zusätzlich kann mit den Rotormagneten 5c und dem Motorstator 6a, 6b eine Vorspannung des Rotors 5 zum Auslassbereich 4 eingestellt werden.

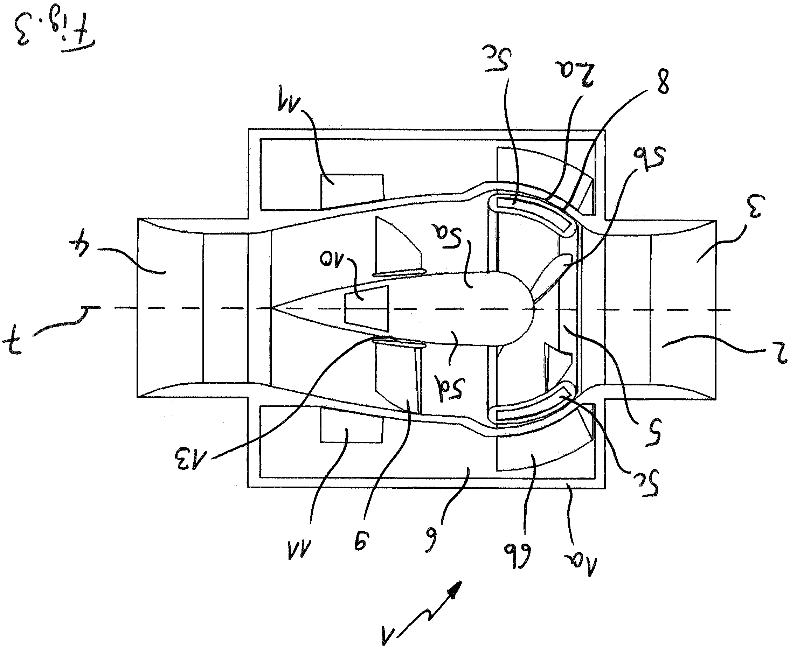

Figur 3 zeigt eine Blutpumpe 1 gemäß einem dritten Ausführungsbeispiel der vorliegenden Erfindung in einer Längsschnittansicht. Die Blutpumpe 1 ähnelt der Blutpumpe aus Figur 2 bis auf die Ausgestaltung des Motorstators 6b und ein zusätzliches passives, magnetisches Statorkipplager 11. Der Motorstator 6b ist bevorzugt eisenlos. Das zusätzliche magnetische Statorkipplager 11 befindet sich im dem Auslassbereich 4 zugewandten hinteren Bereich der Blutpumpe 1 und wirkt mit den Rotormagneten 10 zusammen, welche im tropfenförmigen Element 5d des Rotors 5 angeordnet sind. Das Statorkipplager 11 ist radial abstoßend und hält so das tropfenförmige Element 5d zentral im Fluidkanal 2, wobei verbleibende Kippfreiheitsgrade stabilisiert werden.

Die weiteren gezeigten Merkmale der Blutpumpe 1 stimmen mit der Blutpumpe aus Figur 2 überein. Im sphärischen Abschnitt 2a des Fluidkanals 2 ist der ringförmige Bereich des Rotors 5 auf der zur Kanalwand 2b hin gelegenen Außenseite ebenfalls sphärisch geformt und überstreicht bei Rotation des Rotors 5 eine sphärische Rotationsfläche. Eine Krümmung der Außenseite des ringförmigen Bereichs des Rotors 5 ist an eine Krümmung der Kanalwand 2b angepasst. Weiterhin fallen der Mittelpunkt der sphärischen Krümmung der Außenseite des ringförmigen Bereichs des Rotors 5 und der Mittelpunkt der sphärischen Krümmung des sphärischen Abschnitts 2a im Wesentlichen mit dem Drehpunkt des Lagers 8 zusammen, sodass der minimale Abstand zwischen dem ringförmigen Bereich des Rotors 5 und der Kanalwand 2b auch bei einer Verkippung des Rotors 5 im Wesentlichen konstant ist. Dadurch wird eine Berührung des Rotors 5 mit der Kanalwand 2b und eine damit verbundene mögliche Beschädigung des Rotors 5 und Beeinträchtigung der Funktion der Blutpumpe 1 verhindert.

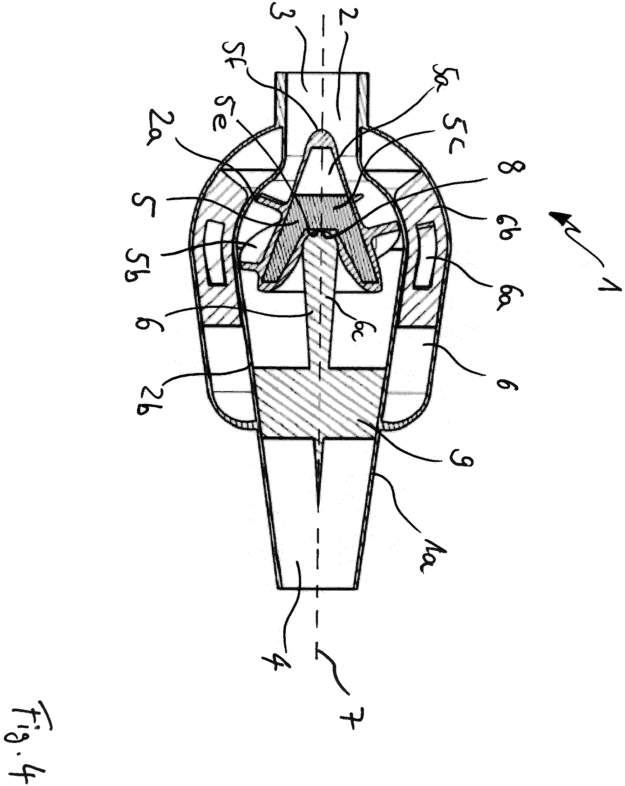

Figur 4 zeigt eine Blutpumpe 1 gemäß einem vierten Ausführungsbeispiel der vorliegenden Erfindung in einer Längsschnittansicht. Die Blutpumpe 1 ähnelt vom Prinzip her der Blutpumpe aus Figur 1. Das hohlzylinderförmige Pumpengehäuse 1a weist einen Einlassbereich 3 und einen Auslassbereich 4 für das Blut auf. Zwischen dem Einlassbereich 3 und dem Auslassbereich 4 führt ein Fluidkanal 2, welcher von einer Kanalwand 2b begrenzt wird. Stromabwärts des Einlassbereichs 2 weist der Fluidkanal 2 einen sphärischen Abschnitt 2a auf und verjüngt sich dann tropfenförmig zum Auslassbereich 4.

Im Wesentlichen unterscheidet sich die Blutpumpe 1 von der Blutpumpe aus Figur 1 in der Form des Rotors 5. Der Rotorkörper 5a des Rotors 5 weist eine Kegelform auf, die an der dem tropfenförmigen Element 6c des Stators 6 zugewandten Seite eine Aussparung 5e aufweist. In diese Aussparung 5e greift das tropfenförmige Element 6c des Stators 6 und bildet somit das axiale und radiale mechanische Ball-Cup-Lager 8 für den Rotor 5. Der Rotorkörper 5a ist derart im Fluidkanal 2 positioniert, dass eine Spitze 5f der Kegelform des Rotorkörpers 5a stromaufwärts des sphärischen Abschnitts in den Einlassbereich 3 hineinragt. Ein Großteil einer Mantelfläche der Kegelform liegt im sphärischen Abschnitt 2a des Fluidkanals 2 und verläuft quasi parallel zur Kanalwand 2b.

Auf der Mantelfläche des Rotorkörpers 5a sind im sphärischen Bereich Förderelemente 5b angeordnet, welche das Blut vom Einlassbereich 3 in Richtung des Auslassbereichs 4 in einer gemischt radialen und axialen Richtung befördern. Die Förderelemente 5b sind derart auf der Mantelfläche angeordnet und derart geformt, dass diese bei Rotation des Rotors 5 eine sphärische Rotationsfläche überstreichen. Weiterhin sind die Förderelemente 5b derart geformt und im Fluidkanal 2 positioniert, dass der Mittelpunkt der sphärischen Rotationsfläche und der Mittelpunkt des sphärischen Abschnitts 2a des Fluidkanals 2 im Wesentlichen mit dem Drehpunkt des Lagers 8 zusammenfallen. Diese Ausgestaltung von Rotor 5 und Fluidkanal 2 ermöglicht, dass ein minimaler Abstand zwischen Rotor 5 und Kanalwand 2 auch bei Verkippung des Rotors 5 konstant bleibt und verhindert, dass bei der Verkippung des Rotors 5 der Rotor 5 die Kanalwand 2b berührt und es zu einer Beschädigung des Rotors 5 oder der Kanalwand 2b bzw. der Beeinträchtigung der Funktion der Blutpumpe 1 kommt.

Die Blutpumpe 1 weist ähnlich wie die Blutpumpe der Figur 1 stromabwärts des Rotors 5 angeordnete Nachleitschaufeln 9 auf, welche das tropfenförmige Element 6c starr mit dem Pumpengehäuse 1a verbinden. Die Nachleitschaufeln 9 unterstützen und verbessern eine Strömung des Blutes zum Auslassbereich 4. Möglicherweise notwendige Spülbohrungen zur Ausspülung des mechanischen Lagers sind nicht gezeigt, können jedoch leicht in den Rotor eingebracht werden.

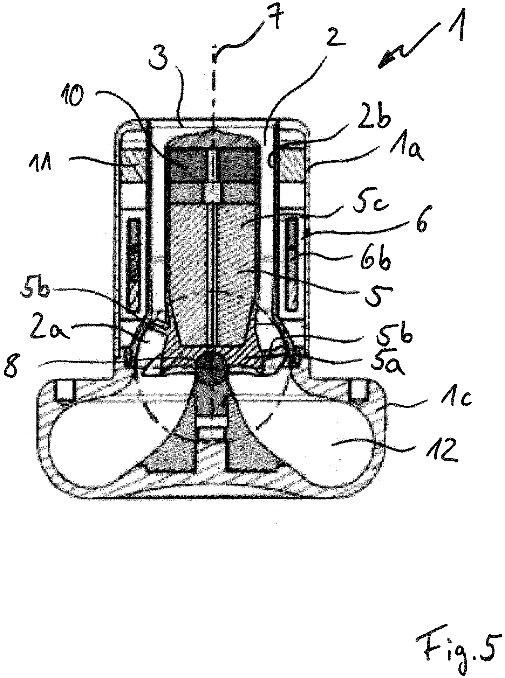

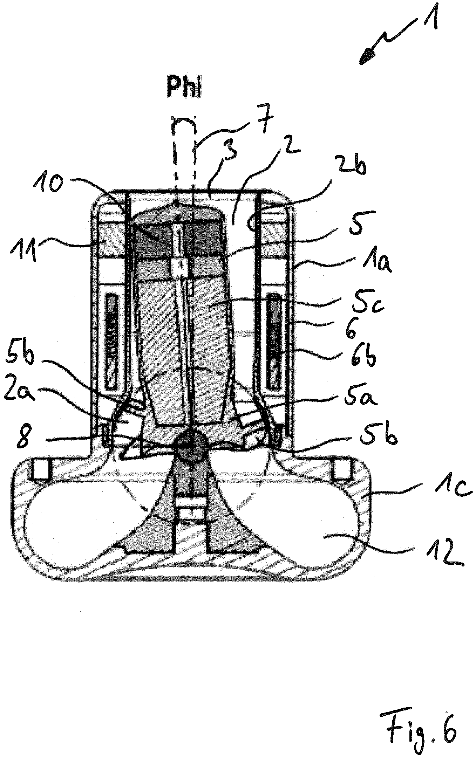

Figuren 5 und 6 zeigen eine Blutpumpe 1 gemäß einem fünften Ausführungsbeispiel der vorliegenden Erfindung in Längsschnittansichten. Die Blutpumpe 1 weist ein im Wesentlichen hohlzylinderförmiges Pumpengehäuse 1a mit einem Einlassbereich 3 und einem Auslassbereich (hier nicht gezeigt) auf, durch welches ein Fluidkanal 2 vom Einlassbereich 3 zum Auslassbereich führt. Der Fluidkanal 2 wird von einer Kanalwand 2b begrenzt und weist zum Auslassbereich hin einen sphärischen Abschnitt 2a auf. Stromabwärts des sphärischen Bereichs 2a weist das Pumpengehäuse 1a eine flanschartige Verbreiterung 1c auf, in welcher der Fluidkanal 2 in eine Ringvolute 12 mündet. Der Auslassbereich (hier nicht gezeigt) befindet sich in der Ringvolute 12.

In dem Fluidkanal 2 ist ein Rotor 5 mit einem im Wesentlichen zylinderförmigen Rotorkörper 5a um eine Drehachse 7 drehbar gelagert. Zur Lagerung des Rotors 5 weist die Blutpumpe 1 ein radiales und axiales mechanisches Ball-Cup-Lager 8 auf, welches im Mittelpunkt der sphärischen Krümmung des sphärischen Bereichs 2a angeordnet ist. Im mittleren Bereich des Rotorkörpers 5a sind Rotormagnete 5c angeordnet, welche mit einem im Pumpengehäuse 1a angeordneten und die Rotormagneten 5c umlaufenden Motorstator 6b zusammenwirken, um den Rotor 5 anzutreiben. Zum Einlassbereich 3 hin gelegen ist in dem Pumpengehäuse 1a ein magnetisches Kipplager 11 angeordnet, welches mit einem im Rotorkörper 5a benachbart zum Kipplager 11 angeordneten Lagermagneten 10 zusammenwirkt, um Kippfreiheitsgrade des Rotors 5 im vorderen, einlassseitigen Bereich der Blutpumpe 1 zu stabilisieren.

Im sphärischen Abschnitt 2a weist der Rotor 5 Förderelemente 5b außen am Rotorkörper 5a auf, welche eine Förderung des zu fördernden Blutes in einer gemischt radialen und axialen Richtung vom Einlassbereich 3 in die Ringvolute 12 ermöglichen. Die Ringvolute 12 ändert dann eine Strömungsrichtung des Blutes von der gemischt radialen und axialen Richtung in eine rein radiale Richtung, bevor das Blut in den in der Ringvolute 12 angeordneten Auslassbereich gelangt. Die Förderelemente 5b weisen ferner eine der sphärischen Krümmung des sphärischen Bereichs 2a entsprechende sphärischen Außenkontur auf, sodass sich bei Rotation des Rotors 5 eine von den Förderelementen 5b überstrichene Rotationsfläche ergibt. Dabei korrespondiert die Krümmung dieser Rotationsfläche mit der Krümmung des sphärischen Bereichs. Ferner sind die Förderelemente 5b so am Rotorkörper 5a positioniert, dass der Mittelpunkt der sphärischen Rotationsfläche und der Mittelpunkt des sphärischen Bereichs 2a im Wesentlichen mit dem Drehpunkt des Lagers 8 zusammenfallen. Diese Ausgestaltung von Rotor 5 und Fluidkanal 2 ermöglicht, dass ein minimaler Abstand zwischen Rotor 5 und Kanalwand 2 auch bei Verkippung des Rotors 5 konstant bleibt und verhindert, dass bei der Verkippung des Rotors 5 der Rotor 5 die Kanalwand 2b berührt und es zu einer Beschädigung des Rotors 5 oder der Kanalwand 2b bzw. der Beeinträchtigung der Funktion der Blutpumpe 1 kommt.

In Figur 6 ist eine Stellung der Förderelemente 5b bei einer maximal möglichen Verkippung des Rotors 5 um eine zur Drehachse 7 orthogonale Achse um einen kleinen Winkel Phi in Bezug auf die Drehachse 7 gezeigt. In diesem gezeigten Fall schmiegt sich der vordere einlassseitige Bereich des Rotors 5 an die Kanalwand 2b an. Dies führt jedoch aufgrund der sphärischen Anordnung der Förderelemente 5b im sphärischen Bereich 2a nicht zu einem Kontakt des Rotors 5, insbesondere der Förderelemente 5b, mit der Kanalwand 2b. Beschädigungen werden somit durch die erfindungsgemäße Ausgestaltung von Rotor 5 und Fluidkanal 2 verhindert und eine dadurch bedingte Beeinträchtigung der Funktion der Blutpumpe 1 vermieden.

Um ein Anschmiegen über einen größeren Bereich zu ermöglichen, können sowohl Rotor 5 und/oder die Kanalwand 2b in diesem Bereich mit einer Abschrägung versehen sein. Des Weiteren sind hydraulisch wirksame Strukturen im Bereich der Rotor-Kanalwand-Berührung denkbar, die eine Auslenkung des Rotors 5 begrenzen oder für zusätzliche, hämokompatible Abstützung sorgen, oder die den entstehenden Vordrall der Strömung im Einlassbereich 3 verstärken oder definieren.

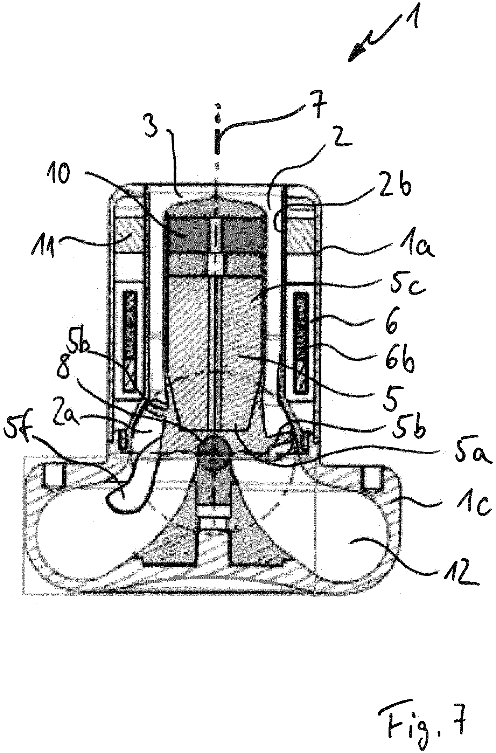

Figur 7 zeigt eine Blutpumpe 1 gemäß einem sechsten Ausführungsbeispiel der vorliegenden Erfindung in einer Längsschnittansicht. Die Blutpumpe 1 ist wie die Blutpumpe in Figuren 5 und 6 aufgebaut und unterscheidet sich lediglich durch das Vorhandensein von in die Ringvolute 12 verlängerten Förderelementen 5f. Die Förderelemente 5f sind wie die anderen Förderelemente 5b zumindest teilweise im sphärischen Bereich 2a des Fluidkanals 2 an einer Außenseite des Rotorkörpers 5a angeordnet. Die Förderelemente 5f sind ferner zur Volute 12 hin am Rotorkörper 5a angeordnet. In den Bereichen der verlängerten Förderelemente 5f, welche sich im Wesentlichen im sphärischen Bereich 2a befinden, ist eine Außenkontur der Förderelemente 5f im Wesentlichen sphärisch geformt, sodass auch diese Bereiche der Förderelemente 5f bei Rotation des Rotors 5 eine sphärische Rotationsfläche überstreichen, wodurch es bei einer Verkippung des Rotors 5 nicht zu einer Berührung des Rotors 5 mit der Kanalwand 2 im sphärischen Abschnitt 2a kommt. Die verlängerten Förderelemente 5f verbessern eine Strömung des Blutes vom Fluidkanal 2 in die Ringvolute 12 und weiter zum Auslassbereich (hier nicht gezeigt) und unterstützen den Wechsel der Strömungsrichtung in der Ringvolute 12 von einer gemischt radialen und axialen Richtung zu einer rein radialen Strömungsrichtung.

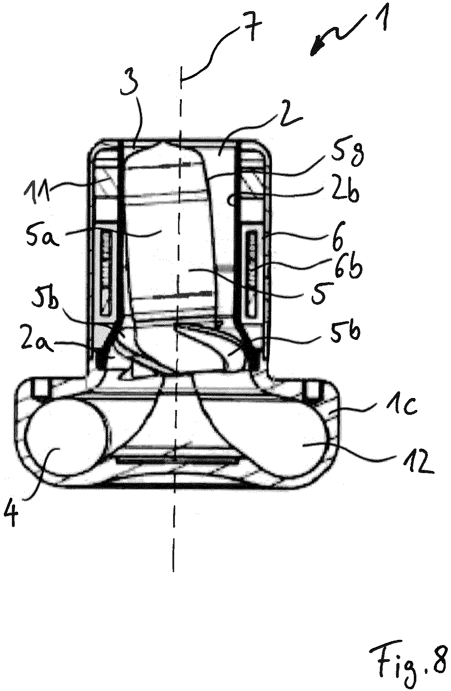

Figur 8 zeigt eine Blutpumpe 1 gemäß einem siebten Ausführungsbeispiel der vorliegenden Erfindung in einer Längsschnittansicht. Die Blutpumpe 1 in Figur 8 ist im Wesentlichen wie die Blutpumpe der Figuren 5 bis 6 aufgebaut und unterscheidet sich lediglich in der Form des Rotorkörpers 5a. Der Rotorkörper 5a weist einlassseitig einen konischen Bereich 5g auf, der sich zum Einlassbereich hin verjüngt und bei maximal möglicher Verkippung des Rotors 5 parallel zur Kanalwand 2b liegt. Dies verbessert ein Anschmiegen des Rotors 5 im einlassseitigen Bereich der Blutpumpe 1 und vermindert zusätzlich die Gefahr einer Beschädigung des Rotors 5 oder der Kanalwand 2b im Falle eines Verkippens des Rotors 5.

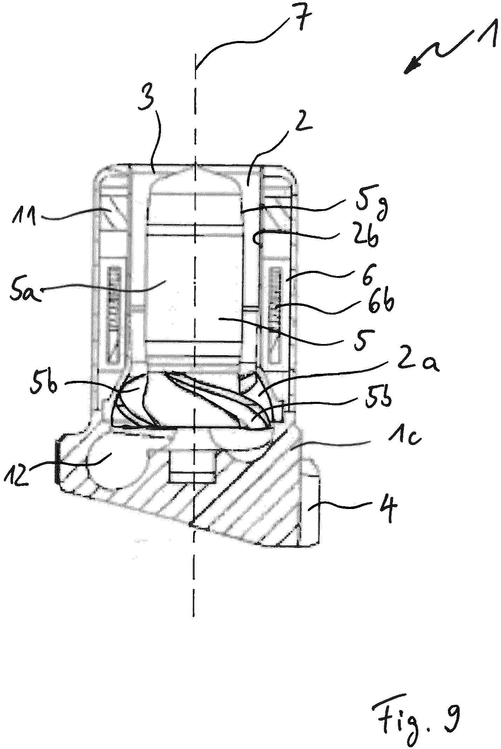

Figur 9 zeigt eine Blutpumpe 1 gemäß einem achten Ausführungsbeispiel der vorliegenden Erfindung in einer Längsschnittansicht. Die Blutpumpe 1 in Figur 8 ist im Wesentlichen wie die Blutpumpe in Figur 8 aufgebaut und unterscheidet sich lediglich in der Form der Volute 12. Die Blutpumpe 1 weist eine semiaxiale Volute 12 auf, welche sehr kompakt das Blut sowohl radial als auch axial bis zum Auslassbereich 4 führt. In der semiaxialen Volute 12 wird das geförderte Blut keinem oder nur sehr langsam einem Wechsel der Strömungsrichtung von einer gemischt radialen und axialen Richtung auf eine rein radiale Strömungsrichtung im Auslassbereich unterworfen. Dies verbessert zusätzlich die Strömung des geförderten Blutes bis zum Auslassbereich 4 verglichen mit einer radialen Ringvolute wie sie beispielsweise in Figur 8 gezeigt ist.

## Patentansprüche

1. Fluidpumpe (1) zum Fördern eines Fluids, insbesondere von Blut, umfassend:
einen Fluidkanal (2), welcher von einer Kanalwand (2b) begrenzt wird,
einen in dem Fluidkanal (2) angeordneten Rotor (5), welcher um einen Drehpunkt des Lagers (8) drehbar mittels eines mechanischen, hydrodynamischen und/oder hydrostatischen, axialen und radialen Lagers (8) gelagert ist, wobei
der Fluidkanal (2) einen sphärischen Abschnitt (2a) aufweist,
der Rotor (5) einen Rotorkörper (5a) und ein daran innerhalb des sphärischen Abschnitts (2a) des Fluidkanals (2) angeordnetes Förderelement (5b) aufweist, welches geeignet ist, eine zumindest bereichsweise im Wesentlichen sphärische Rotationsfläche des Rotors (5) zu erzeugen, und wobei
der sphärische Mittelpunkt des sphärischen Abschnitts (2a) des Fluidkanals (2) und der sphärische Mittelpunkt der sphärischen Rotationsfläche mit dem Drehpunkt im Wesentlichen zusammenfallen, sodass ein minimaler Abstand zwischen dem Rotor (5) und der Kanalwand (2b) im sphärischen Abschnitt (2a) bei Verkippen des Rotors (5) konstant ist.

2. Fluidpumpe (1) nach dem vorhergehenden Anspruch, wobei das Lager (8) ein Ball/Cup-Lager oder ein Pin-Lager, insbesondere ein Nadel-auf-Sackloch-Lager, ist.

3. Fluidpumpe (1) nach einem der vorhergehenden Ansprüche, weiter umfassend ein passiv magnetisches Lager (11), wobei das passiv magnetische Lager als Kipplager zur Rückstellung einer Verkippung des Rotors (5) ausgebildet ist und/oder eingerichtet ist, den Rotor (5) in Bezug auf den Fluidkanal (2) axial vorzuspannen.

4. Fluidpumpe (1) nach einem der vorhergehenden Ansprüche, weiter umfassend einen an der Kanalwand (2b) des Fluidkanals (2) angeordneten Motorstator (6b) und einen im Rotorkörper (5a) oder im Förderelement (5b) integrierten Motormagneten (5c), wobei eine passiv magnetische Kipplagerung durch eine magnetische Anziehung oder Abstoßung zwischen dem Motorstator (6b) und dem Motormagneten (5c) realisiert wird.

5. Fluidpumpe (1) nach einem der vorhergehenden Ansprüche, wobei der Fluidkanal (2) auf einer dem Lager (8) gegenüberliegenden Seite konisch, sich zum Lager (8) hin verjüngend geformt ist, wobei ein Verjüngungswinkel des Fluidkanals (2) einem maximalen Verkippungswinkel des Rotors (5) innerhalb des Fluidkanals (2) entspricht.

6. Fluidpumpe (1) nach einem der Ansprüche 1 bis 4, wobei der Rotorkörper (5a) auf einer dem Lager (8) gegenüberliegenden Seite konisch, sich vom Lager (8) weggerichtet verjüngend geformt ist, wobei ein Verjüngungswinkel des Rotorkörpers (5a) einem maximalen Verkippungswinkel des Rotors (5) innerhalb des Fluidkanals (2) entspricht.

7. Fluidpumpe (1) nach einem der vorhergehenden Ansprüche, weiter umfassend ein im Fluidkanal (2) angeordnetes hydrostatisches oder hydrodynamisches Hilfslager (13) zur Begrenzung der Verkippung des Rotors (5).

8. Fluidpumpe (1) nach dem vorhergehenden Anspruch, wobei das hydrostatische oder hydrodynamische Hilfslager (13) von an der Kanalwand (2b) angeordneten Nachleitschaufeln (9) gebildet wird.

9. Fluidpumpe (1) nach einem der vorhergehenden Ansprüche, wobei auf einer dem Lager (8) gegenüberliegenden Seite des Fluidkanals (2) an der Kanalwand (2b) und/oder am Rotor (5) hydrodynamisch wirksame Elemente angeordnet sind, um bei Verkippung des Rotors (5) ein Anschmiegen des Rotors (5) an die Kanalwand (2b) zu verbessern.

10. Fluidpumpe (1) nach einem der vorhergehenden Ansprüche, wobei der Fluidkanal (2) einen Fluideinlass (3) und einen Fluidauslass (4) aufweist, und das Lager (8) am Fluideinlass (3), am Fluidauslass (4) oder in einer Mitte des Fluidkanals (2) angeordnet ist.

11. Fluidpumpe (1) nach einem der vorhergehenden Ansprüche, wobei der Fluidkanal (2) einen axialen, tangentialen oder axial-tangential-gemischten Fluidauslass (4) aufweist.

12. Fluidpumpe (1) nach einem der vorhergehenden Ansprüche, wobei der Fluidkanal (2) einen Fluidauslass (4) umfasst, und die Fluidpumpe (1) im Bereich des Fluidauslasses (4) eine Volute (12) aufweist, insbesondere eine Ringvolute, eine logarithmische Volute und/oder eine Volute mit einem axialen Anteil.

13. Fluidpumpe (1) nach einem der vorhergehenden Ansprüche, wobei das mechanische Lager (8) ein hämokompatibles, hartes, abriebfestes und/oder wärmeleitfähiges Material, insbesondere Keramik, insbesondere Aluminiumoxid (Al₂O₃), Siliziumcarbid (SiC), Zirkoniumoxid (ZrO₂) oder Siliziumnitrid (Si₃N₄), Mischkeramik, insbesondere Al₂O₃/SiC, Aluminium-verstärktes Zirkoniumoxid (ATZ) oder Zirkoniumoxid-verstärktes Aluminiumoxid (ZTA), Kristallin, insbesondere Diamant, Saphir, Rubin oder Quarz, oder Tantalnitrid, insbesondere eine Tantalnitrid-Dünnschicht aufweist oder daraus besteht, und/oder eine Gleitschicht, insbesondere Diamond-Like Carbon (DLC), SiN oder Wolframcarbid/Kohlenstoff (WC/C) aufweist.

14. Fluidpumpe (1) nach einem der vorhergehenden Ansprüche, wobei das Lager (8) axial mechanisch verschiebbar ist, um einen idealen Abstand zwischen dem Fluidkanal (2) und dem Förderelement (5b) einzustellen und/oder wobei das magnetische Lager (11) und/oder der Motorstator (6b) axial verschiebbar ist, sodass die Vorspannung des Rotors (5) im Lager (8) einstellbar ist.

15. Fluidpumpe (1) nach dem vorhergehenden Anspruch, wobei das Lager (8) vor Inbetriebnahme der Fluidpumpe (1) mittels eines Gewindes axial mechanisch verschiebbar ist.
